# EUROPEAN PATENT SPECIFICATION

(11) **EP 2 750 601 B1**
(45) Date of publication and mention of the grant of the patent: **17.08.2016**
(21) Application number: 12781419.2
(22) Date of filing: 30.08.2012
(51) Int. Cl.: A43B 13/38, B29D 35/00, A61B 5/11

(54) **DEVICE FOR MONITORING BALANCE AND A METHOD FOR MANUFACTURING THEREOF**
VORRICHTUNG ZUR GLEICHGEWICHTSBEOBACHTUNG UND VERFAHREN ZU IHRER HERSTELLUNG
DISPOSITIF PERMETTANT DE SURVEILLER LE MAINTIEN DE L'ÉQUILIBRE ET PROCÉDÉ DE FABRICATION DUDIT DISPOSITIF

(30) Priority: 30.08.2011 HU P1100471
(43) Date of publication of application: 09.07.2014
(73) Proprietor: Bay Zoltán Alkalmazott Kutatási Közhasznú Nonprofit KFT., 1116 Budapest (HU)
(72) Inventor: VASS, Dezso, H-3713 Arnót (HU); ÁRVAI, László, H-3532 Miskolc (HU)
(74) Representative: Kereszty, Marcell
(86) International application number: PCT/HU2012/000082
(87) International publication number: WO 2013/038214

(56) References cited:
- WO-A1-2010/088696
- DE-A1- 10 201 134
- US-A1- 2009 216 156

## Description

### TECHNICAL FIELD

The invention relates to a device for monitoring balance and a method for manufacturing the device.

### DESCRIPTION OF PRIOR ART

Monitoring balance of patients is generally carried out by means of devices comprising a force plate or a balance board. These devices are usually installed at healthcare institutions. Before starting the test, the patient steps on the device, holds his/her arms straight out in front of him/her parallel with the ground, closes his/her eyes, and stands in this posture for a few 10 seconds trying to keep his/her balance as stable as possible. The measurement supplies exact results but reflects only a short-time status and is not capable of giving information of the gait of the patient and his/her balance during walking. A number of methods are known for recording patients' gait parameters that can be grouped into contact and no contact (e.g. applying cameras) methods. The most Widespread contact solutions are sole pressure distribution measurement (optical or resistive) methods and the application of MEMS (Micro-Electro-Mechanical Systems) sensors. Instruments applied for these measurements usually comprise wired connections, and may partially be integrated in patient's footwear.

CN 101869481 A discloses a shoe insole-like device that measures the pressure exerted on the shoe insole by the sole of the foot. Measurement is performed utilizing pressure sensors disposed between metal-based carrier plates. The device is only capable of measuring body weight, and does not supply any information on the centre of gravity of the body. The applied metal plates make the insole rigid, and therefore it is uncomfortable to wear for a prolonged period. Since the device does not comprise any further sensors, it cannot give information on gait parameters such as step length.

The homepage of Tekscan, Inc. (http://www.tekscan.com/medical/gait-analysis.html) presents devices for gait analysis. These products, although they are adapted to be placed inside footwear, do not operate as an independent unit. The device requires an ankle strap that transfers the signals of a pressure sensor matrix placed inside footwear to a processing unit. The above described devices are not capable of gait analysis under home care conditions.

US 2009/0137933 A1 discloses a smart shoe insole requiring external connection that is relatively uncomfortable, and has more vulnerable structure. This solution is incapable of static measurement, and can be only applied for dynamic measurements (performed during walking). The device comprises two pressure sensors for measuring sole pressure. The insole also comprises an acceleration sensor, but for recording gait parameters it applies high power-consumption GPS technology instead of gyroscopes.

US 6,122,960 discloses a device that measures height and distance of steps. The description tangentially mentions the possibility of increasing accuracy utilizing a neural network. The device is not suitable for monitoring balance.

DE 102 01 134 A1 discloses a shoe insole suitable for measuring dynamic forces.

### DISCLOSURE OF THE INVENTION

An objective of the present invention is to provide a device that reduces or eliminates the drawbacks of prior art solutions to the greatest possible extent. A further objective of the invention is to provide a device that operates efficiently, supplies accurate results, and has low production costs. It is also an objective of the invention to provide a device that allows the monitoring of balance during walking in an easy and efficient manner. A further objective is to provide an efficient method for manufacturing and calibrating (training) the device.

The objectives according to the present invention have been achieved by providing a method according to claim 1, and a device according to claim 5. Preferred embodiments of the invention are defined in the dependent claims.

### BRIEF DESCRIPTION OF THE DRAWINGS

Preferred embodiments are described in the following with reference to the accompanying drawings, in which
Fig.1 is a schematic plan view of a device implemented as a smart shoe insole,
Fig. 2 shows a schematic side view of the device shown in Fig. 1,
Fig. 3 is a schematic bottom view of the device shown in Fig. 1,
Fig. 4 shows a block diagram of a portion of the device shown in Fig. 1,
Fig. 5 illustrates how functional units necessary for operating the device may be arranged on the body of a patient,
Fig. 6 is a schematic drawing illustrating the operation of the device,
Fig. 7 is a functional diagram of the evaluation and training process of the device,
Fig. 8 is a schematic view of a preferred embodiment of the device, showing a pressure pin applied for calibration,
Fig. 9 shows a schematic view of Fig. 8, completed with the guide plates for the pressure pins, and
Fig. 10 shows a perspective view of a pressure pin arrangement applied for calibration, together with the device.

### DETAILED DESCRIPTION OF THE PREFERRED EMBODIMENTS

The invention relates to a device suitable for the detection of a state of balance, and preferably the monitoring of gait parameters. The device is implemented as a shoe insole that may be placed inside footwear also supporting the heel of the foot (such as shoes, sandals, slippers). The device is implemented as a measurement unit providing integrated measurement and communication functions that, thanks to its special materials and configuration (shape), its built-in sensors (strain gauges, gyroscope, acceleration and temperature sensors) and evaluation algorithm, is able to continuously monitor balance, body posture, and preferably also the gait parameters of its user. Preferably, the device has a completely self-enclosed configuration, its batteries may be recharged without galvanic connection. The device may be applied for assisting in home care and surveillance, as well as in condition survey of patients with certain diseases and conditions such as Parkinson's disease, sclerosis multiplex, and stroke rehabilitation.

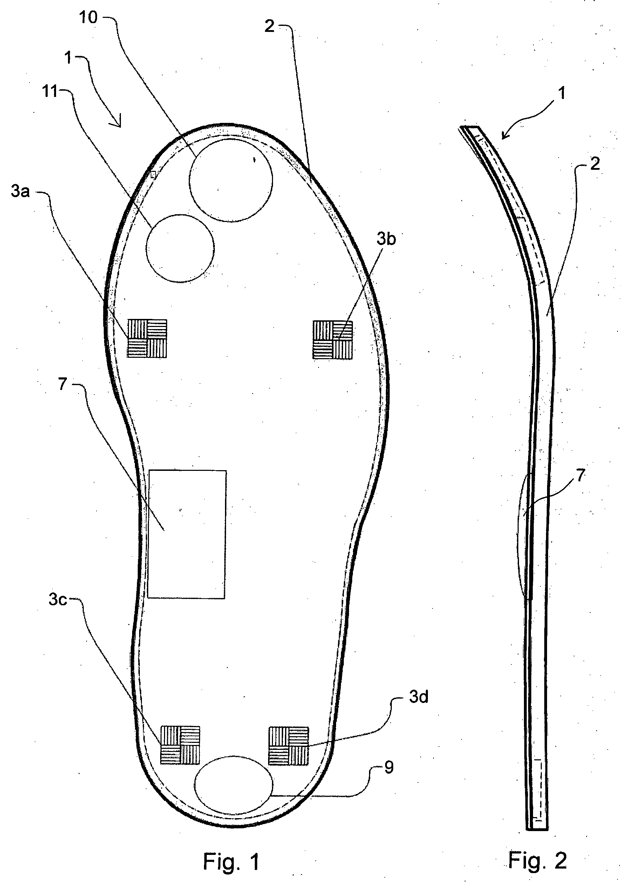

Fig. 1 is a plan view of a preferred embodiment of the device, clearly showing the major functional parts and their arrangement. A carrier plate 1 of the device is sized to correspond to the shoe size of the user, and is shaped as a shoe insole.

The carrier plate 1 is made of a composite material, providing a lightweight, flexible, and bendable configuration. Our experiments have shown that it is preferable to apply at least three layers of fibreglass fabric, each layer being rotated by 90 degrees with respect to the other and embedded in synthetic resin. The composite material is prepared with a mould shaped like a shoe insole. The recommended drying and curing period is 5 days.

After polishing and cleaning the surface of the carrier plate 1, a flexible support that is required for measurements is prepared. A flange 2, made preferably from silicon, is formed along the circumference of the carrier plate 1 to provide flexible support for the carrier plate 1. As indicated by experimental results, the support provided by the flange allows high flexibility and therefore high measurement sensitivity. The flange 2 may in specific cases be formed in a discontinuous manner instead of extending along the entire circumference of the carrier plate 1, while specific support means may also be provided at other points or regions thereof. It may also be conceived that the entire undersurface of the carrier plate 1, or larger portions of the undersurface are covered with a flexible support layer. Such embodiments allow a relatively lower surface deformation to occur under a given force than the embodiment comprising only a support flange. However, the circumferentially arranged flexible support portions constitute a support flange 2 also in these cases.

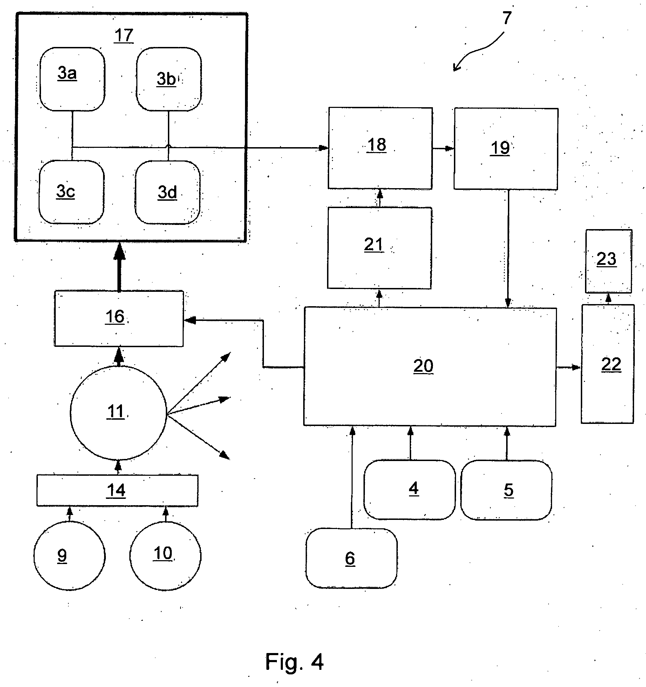

In a preferred arrangement, four strain gauges 3a, 3b, 3c, 3d are fixed by adhesive bonding to the carrier plate 1 at locations conforming to the anatomy of the foot and exceptionally preferable for measuring balance. The term "strain gauge" refers preferably to a special, commercially available arrangement where four strain gauges are arranged on a single "stamp" (carrier body) such that one pair of strain gauges is arranged at 90° with respect to the other pair, the identically oriented gauges being arranged transversely to one another. This special strain gauge arrangement is applied because it allows the measurement of x and y-direction deformation on a single "stamp" (carrier body) such that the bridge also performs temperature compensation due to the two pairs of identically oriented strain gauges. The strain gauges 3a, 3b, 3c, 3d therefore constitute individual measuring bridges 17 that have their outputs connected to the input of a measurement amplifier 18 (Fig. 4). The strain gauges 3a-d provide the input signals required by the system for determining a centre of gravity of a body. As the centre of gravity gets displaced, the strain gauges 3a-d are stretched on one side and compressed on the other side. Due to the anisotropic behaviour of the composite carrier plate 1, the extent of stretch and compression cannot be adequately described by analytical methods. The curved planar shape and inhomogeneous structure of the plate cause the elastic modulus to be different at each point.

Thanks to its arched structure, the human foot is supported on the ground at only three points: at the first metatarsal bone (front, medial); at the fifth metatarsal bone (front, lateral); and at the calcaneus (rear). As indicated by our experiments, the most exact monitoring may be achieved utilizing the least possible number of strain gauges 3a-d in case the strain gauges 3a-d are arranged as follows: a first strain gauge 3a is arranged within a proximity of 10 mm of the region under the first metatarsal bone, and a second strain gauge 3d is arranged within a proximity of 10 mm of the region under the fifth metatarsal bone, the second strain gauge 3b being arranged symmetrically with the first strain gauge 3a with respect to a midline extending between the two outermost points of the carrier plate 1, while a third strain gauge 3c and a fourth strain gauge 3d are arranged symmetrically with respect to the midline in a region located 5-15 mm towards the heel from the lowest portion of the calcaneus.

A battery 11 and a secondary coil 10 adapted to allow inductive charging are disposed at the end of the carrier plate 1 facing the toes. A piezoelectric plate 9 is arranged in the region towards the heel. As the user walks, the plate 9 becomes loaded by the weight of the body transferred through the heels, producing electric voltage that may be applied for continuous charging of the battery 11 utilizing a charge controller 14.

A central electronic unit 7, to be described in more detail later, is also fixed to the carrier plate 1. The strain gauges and the central electronic unit 7 are connected to each other with conduits that are not shown in the drawings.

Fig. 2 shows a side elevation view of the carrier plate 1. As it can be seen from the drawing, the support flange 2 performs a double role. First, it provides a support for the carrier plate 1 so that it may undergo deformation under pressure, and second, it provides a clearance required for the functional units secured to the carrier plate 1.

The central electronic unit 7 may in specific cases extend to the free space provided by the arch of the foot.

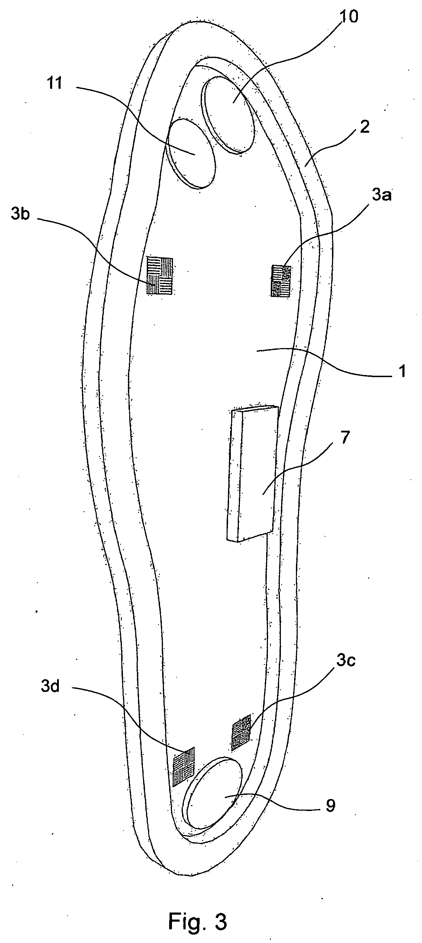

Fig. 3 shows a schematic bottom view of the device shown in Fig. 1. The strain gauges 3a-d that have relatively smaller height are preferably also fixed to the undersurface of the carrier plate 1 by adhesive bonding.

Fig. 4 shows a block diagram of a preferred embodiment of the device. The output signal of the strain gauges 3a-d connected in bridges 17 are fed to a measurement amplifier 18 and subsequently to an analogue-to-digital converter 19, and to a microcontroller 20 responsible for data processing and control. Offset and drift compensation 21 of the measurement amplifier 18 is performed by the microcontroller 20 preferably by software. The central electronic unit 7 performs the following functions: on/off switching of the bridges, calibration and measurement; gyroscope calibration and measurement; acceleration sensor offset calibration and measurement; processing and acquisition of measurement data; power management; communication.

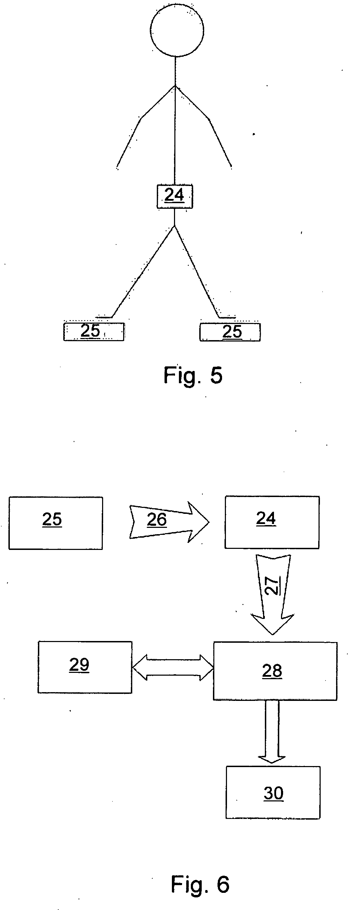

A three-dimensional acceleration sensor 5 is preferably also comprised in the central electronic unit 7, adapted to provide information on the position of the insole and the accelerations generated during walking. This information, supplemented by the signal of the three-dimensional gyroscope 4, allows the determination of gait parameters. A temperature sensor 6 is applied primarily for detecting that the device is being worn (by sensing the heat of the foot of the user), while during battery charging it monitors the temperature of the battery 11, and warns of overcharging. After data processing, measured values are sent by the microcontroller 20 through a wireless connection 26 to a receiver unit 24 utilizing a radio frequency unit 22 and an antenna 23 connected thereto (Fig. 6). The wireless connection 26 also provides bidirectional connection to an inductive charging unit.

The low-power central electronic unit 7 is preferably disposed under the arch of the foot. Flexible support means are formed along the circumference, as well as at other points - preferably specified during manufacturing - of the carrier plate 1. The measurement unit is a sealed, self-enclosed unit having no openings or electric connections on its surface. After the assembly is completed, the area surrounded by the flexible flange 2 may in specific cases be easily filled with flexible adhesive, and/or the entire device may be covered with waterproofing sealing material such that perfect water and moisture proofing, as well as mechanical protection of the structural elements may be provided. The energy required for operating the electronic components is supplied by a built-in battery 11. The charging of the battery 11 can be realized without any galvanic connection, by inductive means with a coil 10. To reduce the size of the battery 11, energy generated from oscillations occurring during walking is recuperated by a piezoelectric plate 9 disposed near the heel. Data acquisition is preferably continuous, but the communication is discontinuous or event-driven to save power. To optimise power consumption the supply of the strain gauges 3a-d is also switched on and off in a controlled manner (for instance, when the patient is sitting it is unnecessary to operate the strain gauges), which is controlled by a bridge power control unit 16 under the control of the microcontroller 20.

Fig. 5 shows an operating position of the device 25. Because of the low power requirements, the wireless communication system of the device 25 only allows a short-distance data connection, it communicates with a separate, independent, self-powered receiver unit 24 attached preferably to a belt or to a clothing of the patient; the receiver unit 24 being able to establish a longer-distance data connection.

As shown in Fig. 6, the device 25 communicates with the receiver unit 24 through a bidirectional wireless connection 26. Besides other possible functions, the receiver unit 24 receives and transmits data over a GSM network 27 through the Internet 28 to a central data acquisition system 29 that may be controlled by medical professional staff through a computer 30.

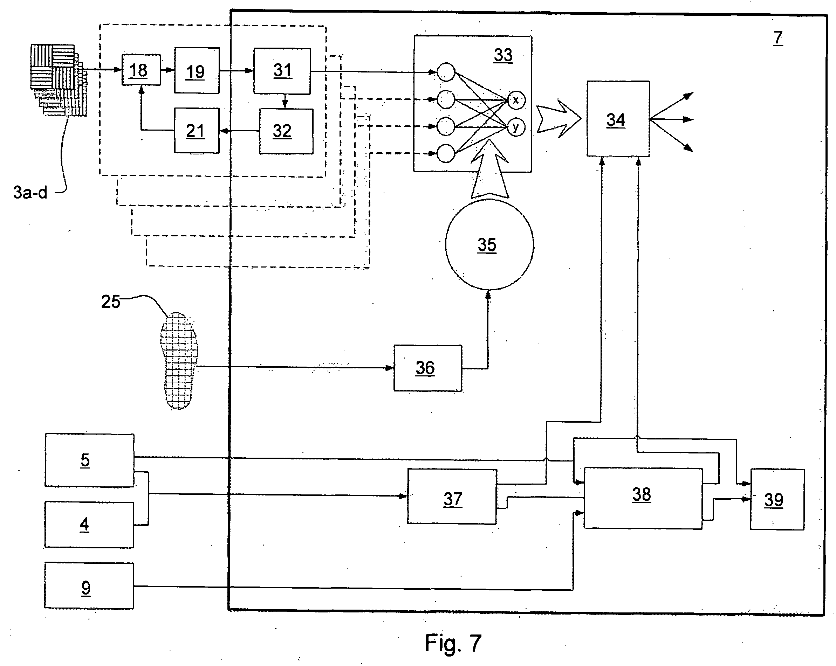

Fig. 7 shows a block diagram of the evaluation and training systems of a preferred embodiment. Each of the four strain gauges 3a-d has an assigned measurement channel, which has a structure as follows: a measurement amplifier 18 receives, amplifies and transfers with offset compensation the signal of the strain gauge 3a-d to an analogue-to-digital converter 19. Digital data are fed from the analogue-to-digital converter 19 to a preprocessing and filtering algorithm 31. The algorithm 31 performs noise filtering, and with an algorithm 32 determining the amplitude and offset of the input signal to control the offset and drift compensation function 21 utilizing a suitable digital to analogue converter. The filtered signals of the four measurement channels are fed to a neural network-based algorithm 33 that outputs x, y coordinates of a centre of gravity (preferably also the force magnitude data) to be fed to a position evaluation algorithm 34. The neural network-based algorithm 33 may be implemented in software or hardware, or in a combination thereof. A neural network-based algorithm 33 for determining the position of the centre of gravity of the body, operating on the basis of signals of the strain gauges, is therefore provided in the central electronic unit 7. As for the output of the balance evaluation algorithm 34 there are three possible states:
- Balance OK
- Instable balance
- Loss of balance

Due to the anisotropic character of the composite carrier plate 1 (the elastic modulus is dependent at every point on the direction and magnitude of load), it is necessary to record the output data (calibration data) of the strain gauges 3a-d under different load values at several points of the carrier plate 1. The calibration data 35 thus obtained are utilised as input data of the training algorithm 36 of the neural network adapted for computing the position of the centre of gravity of the body. The operating and training algorithm of the neural network (feed-forward back propagation) is known *per* se and is described in detail in literature.

Balance may be determined also in certain phases of steps during walking: Specific step phases may be detected and gait parameters, such as step length and step number may be determined utilizing the signals of the inertial measurement system. The inertial measurement system consists of a three-dimensional acceleration sensor 5 on the one hand and on the other hand a three-dimensional gyroscope 4. Euler angles are computed from the signals of the inertial measurement system utilizing a Kalman filter 37. The Kalman filter 37 is applied for eliminating the offset and drift errors of the three-dimensional gyroscope 4, using the data measured by the three-dimensional acceleration sensor 5. The detailed description of the Kalman filter 37 and the Euler angles can be found in the literature. Step detection is performed by an algorithm 38 utilizing the signals of the three-dimensional acceleration sensor 5, with the Euler angles and the signal of the piezoelectric plate 9 applied as supplemental data. The algorithm 39 for computing step length, is based on double integration of accelerations taking into account the step detection results. Signals of the inertial measurement system may also be evaluated together with the signals provided by the strain gauges 3a-d that may especially be useful for computing dynamic balance during walking.

To process the measurement data of the strain gauges 3a-d, a different, unique algorithm is required for each individual carrier plate 1, where the input data is given by the deformation occurring under different loads at different points. The algorithm generates a unique calibration table by "learning" these deformations.

Since the strain gauges measure the deformation of the carrier plate that is dependent on the location of the application point of the force exerted on the insole due to the insole's special shape, it is not possible to determine the magnitude and the application point of the force utilizing a general algorithm.

Due to the limited running resources available in the microcontroller integrated in the insole, the applied algorithm cannot be overly complex. In addition, the algorithm should also be easily adaptable if possible, because of the requirements of different insole sizes and exact fit to the mechanical properties of the given insole.

To determine the application point (and preferably also the magnitude) of the force, a neural network system is provided. The advantage of the neural network systems that they are easily adaptable through "training", which involves the tuning of the internal coefficients of the network. The number of arithmetic operations required for the operation of neural networks is known when the network is designed, and is independent of the input data. Neural algorithms only comprise addition and multiplication as arithmetical operations. A so-called "transfer function" should be generated, which can be performed relatively easily, by creating a simple table.

The so structured neural network, or more exactly, neural network-based algorithm, receives as inputs the filtered, preprocessed (normalized) signals of the strain gauges, and provides on its outputs the coordinates (and preferably also the magnitude) of the force. Before the operation, the neural network should be trained applying suitable measurement data, which practically means determining the optimal values of the internal coefficients (weight factors) of the network.

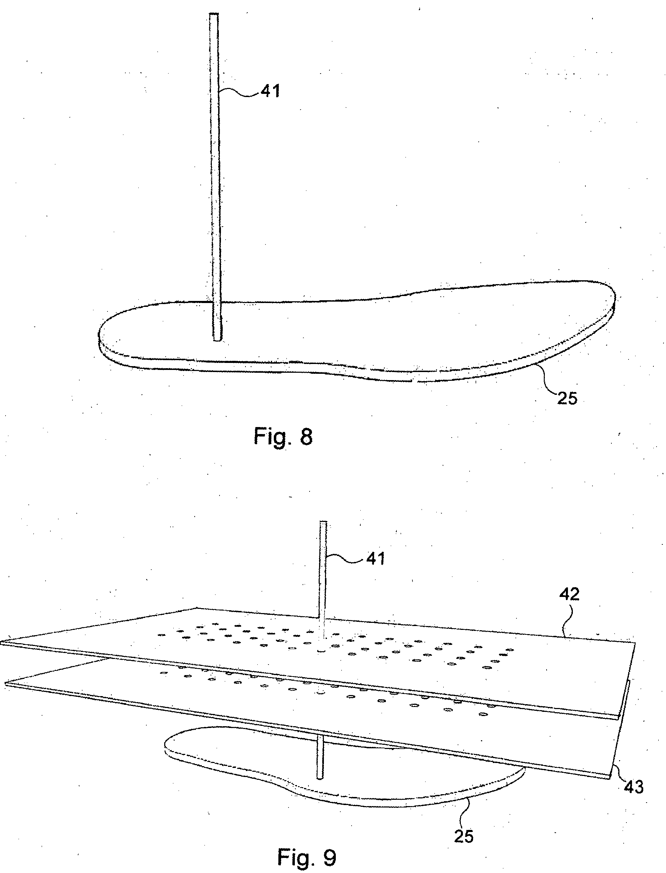

The training process requires deformation data measured on a real insole. Training is performed applying the arrangement illustrated in Fig. 8. The neural network-based algorithm is trained by applying a pressure force or forces to the carrier plate 1 of the device 25 at several places of the plate utilizing a pressure pin or pins 41, measuring the signals of the strain gauges resulting from the deformation caused by the pressure force or forces; and utilizing the thus obtained measurement data as training data for the neural network-based algorithm. As it is illustrated in Fig. 9, the pressure pins 41 are preferably passed through openings in two guide plates 42, 43.

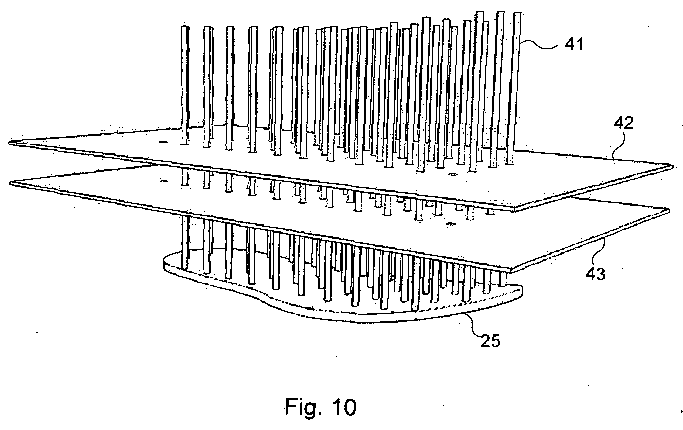

As seen in Fig. 10, pressure pins 41 arranged in a 20x20-mm grid pattern are applied during the calibration process. Pressure force of at least three different magnitudes are exerted one by one on each pressure pin 41, resulting in a deformation of the carrier plate 1 and a change in the resistance of the strain gauges adhesively bonded thereto. Measurement values of the strain gauges 3a-d corresponding to the given pressure force values are preferably recorded for each pressure pin 41. The advantage of training utilizing only one pressure pin 41 at a time is that the coordinates of the centre of gravity corresponding to a given pressure force are the same as the coordinates of the pressure pin 41 exerting the force. This allows a simple training/validation process. It is of course also possible to apply a training process during which more than one pressure pins 41 are applied for exerting pressure forces at any given time. In this case, the centre of gravity corresponding to the combined pressure forces may be computed simply by weighing the measured values. However, due to the anisotropic behaviour of the plate it is important to apply more than one force values at each point for the training operation.

As a result of the measurements, a deformation map showing the deformation responses of the carrier plate 1 to different force values is obtained. After the necessary validation, filtering, and preprocesing (e.g. normalization) operations these data may be applied for training the neural network. Most of the algorithms described in literature are applicable for training. These are multiple-step iteration algorithms that generate the optimal neural network structure (the structure operating with the minimal error) in a gradual manner.

A set of measurement data (generally 5 to 10 percent of data) should be separated for the purposes of utilizing those as so called control data for the training process. These data are removed from the data applied for training, and the response of the network to these data is recorded in each training step. Since in this case not only the input but also the correct (expected) output data are available, the quality of training may be checked. In case the response of the neural network system generates data within an acceptable margin of error, the training process can be regarded as finished.

Because of the anisotropic character of the composite carrier plate 1 it is necessary to record the output signals of the strain gauges corresponding to the deformation response of the carrier plate 1 at several points or regions under different load values (as calibration data). Calibration data are applied as training data of the neural network-based algorithm adapted to compute the centre of gravity of the body, from which the algorithm generates the unique mappings characteristic of each carrier plate 1 determining the centre of gravity of the body (and preferably the magnitude of pressure force).

The invention may be especially preferably applied for the following areas:
in home care, for patient surveillance
   - body posture detection
   - signalling unstable balance
   - fall detection
   - detecting non-motion
   - detection of leaving home
   - status monitoring in Parkinson's disease
gait analysis
   - step length
   - step number
   - displaying relative path
   - freeze detection
   - detecting unstable balance
   - fall detection
telerehabilitation
   - support of physical therapy.

The patient is released home from the hospital or consultation wearing a footwear with a properly sized smart insole and a waist-mounted communication unit. The insole should be placed by the patient inside one of the shoes currently worn. The smart insole performs continuous measurements of balance and of gait parameters, which are transferred to a central data acquisition unit utilizing the waist-mounted data receiver and communication unit. A doctor may yield useful information by evaluating the data of the smart insole, taking into account both current values and trends.

The advantages of preferred embodiments are detailed below.
- The device is capable of continuously and simultaneously measuring gait and balance parameters as long as the patient is wearing the footwear in which the insole is placed, and thereby it becomes possible to monitor the motion and status of the patient during most of the day. In addition to performing measurements, the device may thus be advantageously applied also for patient surveillance (for dizziness and fall detection).
- For determining a state of balance, a system of strain gauges has been created, which are adhesively bonded to a flexible carrier body in a special arrangement. Processing the information thus obtained is, however, a very complex task due to the curved surface and inhomogeneous flexibility of the material. The artificial intelligence-based software developed for data processing also forms a part of the system.
- Integrating the three functions (determining the centre of gravity of the body, determining gait parameters, dizziness and fall detection) in a single shoe insole that has no openings or electric connections on its surface allows for more comfortable, easier use by the patient, since instead of three only one device has to be worn.

The device is a self-enclosed, compact device that contains power supply, data processing, and communications electronic units, and therefore does not require any wired connection to any other unit.

The present invention is not limited to the preferred embodiments presented above, and further modifications and changes may also be possible within the scope defined by the claims.

## Claims

1. Method for manufacturing a device for monitoring balance, comprising the steps of
- providing a carrier plate (1) shaped like a shoe insole,
- fixing strain gauges (3a-d) onto the carrier plate (1),
- fixing a central electronic unit (7) onto the carrier plate (1), and
- connecting the strain gauges (3a-d) and the central electronic unit (7) with each other with conduits,
- forming a flange (2) adapted for providing flexible support to the carrier plate (1) along a circumference of the carrier plate (1), and
- providing in the central electronic unit (7) a neural network-based algorithm (33) for determining a position of a centre of gravity of a body, operating on the basis of signals of the strain gauges (3a-d),
wherein the neural network-based algorithm (33) is trained by
- applying pressure forces to the carrier plate (1) at several places thereof by utilizing pressure pins (41) arranged in a grid pattern for the application of the pressure forces, and exerting pressure forces of at least three different magnitudes with each pressure pin (41),
- measuring the signals of the strain gauges (3a-d) resulting from the deformation caused by the pressure forces; and
- utilizing the thus obtained measurement data as training data for the neural network-based algorithm (33).

2. The method according to claim 1, **characterised by** separating a set of measurement data as control data, where these control data are not applied as training data, but instead the response of the neural network-based algorithm (33) to the control data is tested from time to time during the training process, and finishing the training process when the response generated by the neural network-based algorithm (33) to the control data is within a margin of error.

3. The method according to claim 1, **characterised in that** a three-dimensional acceleration sensor (5) and a three-dimensional gyroscope (4), both adapted for monitoring gait parameters, as well as a temperature sensor (6), are also fixed onto the carrier plate (1).

4. The method according to any of claims 1 to 3, **characterised by** that the entire device is covered with a waterproof sealing material.

5. A device for monitoring balance, comprising
- a carrier plate (1) shaped like a shoe insole,
- strain gauges (3a-d) fixed to the carrier plate (1), and
- a central electronic unit (7) connected with conduits to the strain gauges (3a-d) and fixed to the carrier plate (1),
wherein
- a flange (2) adapted for providing flexible support to the carrier plate (1) is formed along a circumference of the carrier plate (1), and
- the central unit (7) comprises a neural network-based algorithm (33) adapted for determining - on the basis of signals of the strain gauges (3a-d) - a position of a centre of gravity of a body, and being trained according to claim 1.

6. The device according to claim 5, **characterised by** that the strain gauges (3a-d) are arranged in strain gauge units adhesively bonded to the carrier plate (1), wherein each strain gauge unit comprises a carrier body and four strain gauges having identical parameters, the four strain gauges being arranged on the carrier body rotated at 90 degrees with respect to each other.

7. The device according to claim 6, **characterised by** comprising
- a first strain gauge (3a) arranged within a proximity of 10 mm of the region under the first metatarsal bone, and a second strain gauge (3b) arranged within a proximity of 10 mm of the region under the fifth metatarsal bone symmetrically with the first strain gauge (3a) with respect to a midline extending between the two outermost points of the carrier plate (1), and
- a third strain gauge (3c) and a fourth strain gauge (3d) arranged symmetrically with respect to the midline in a region located 5-15 mm towards the heel from the lowest portion of the calcaneus.

8. The device according to any one of claims 5-7, **characterised by** further comprising a three-dimensional acceleration sensor (5) and a three-dimensional gyroscope (4) adapted for monitoring gait parameters, as well as a temperature sensor (6), all fixed to the carrier plate (1).

## Patentansprüche

1. Verfahren zur Herstellung einer Vorrichtung zur Gleichgewichtsüberwachung, das die folgenden Schritte aufweist
- Bereitstellen einer wie eine Schuh-Einlegesohle geformten Trägerplatte (1),
- Befestigen von Dehnungsmessstreifen (3a-d) an der Trägerplatte (1),
- Befestigen einer zentralen Elektronikeinheit (7) an der Trägerplatte (1), und
- Verbinden der Dehnungsmessstreifen (3a-d) und der zentralen Elektronikeinheit (7) miteinander durch Leitungen,
- Formen eines Flanschs (2), der geeignet ist, entlang eines Umfangs der Trägerplatte (1) eine biegsame Stütze für die Trägerplatte (1) zu liefern, und
- Bereitstellen in der zentralen Elektronikeinheit (7) eines auf einem neuronalen Netzwerk basierenden Algorithmus (33) zur Bestimmung einer Stellung eines Schwerpunkts eines Körpers, der auf der Basis von Signalen der Dehnungsmessstreifen (3a-d) arbeitet,
wobei der auf einem neuronalen Netzwerk basierende Algorithmus (33) trainiert wird durch
- Anwenden von Druckkräften an die Trägerplatte (1) an mehreren Stellen von dieser durch Verwendung von in einem Gittermuster angeordneten Druckstiften (41) zur Anwendung der Druckkräfte, und Ausüben von Druckkräften mindestens drei verschiedener Stärken mit jedem Druckstift (41),
- Messen der Signale der Dehnungsmessstreifen (3a-d), die von der durch die Druckkräfte verursachten Verformung stammen; und
- Verwenden der so erhaltenen Messdaten als Trainingsdaten für den auf einem neuronalen Netzwerk basierenden Algorithmus (33).

2. Verfahren nach Anspruch 1, **gekennzeichnet durch** Trennen eines Satzes von Messdaten als Steuerdaten, wobei diese Steuerdaten nicht als Trainingsdaten angewendet werden, sondern stattdessen die Antwort des auf einem neuronalen Netzwerk basierenden Algorithmus (33) auf die Steuerdaten von Zeit zu Zeit während des Trainingsvorgangs getestet wird, und Beenden des Trainingsvorgangs, wenn die von dem auf einem neuronalen Netzwerk basierenden Algorithmus (33) erzeugte Antwort auf die Steuerdaten sich innerhalb einer Fehlertoleranz befindet.

3. Verfahren nach Anspruch 1, **dadurch gekennzeichnet, dass** ein dreidimensionaler Beschleunigungssensor (5) und ein dreidimensionales Gyroskop (4), die beide zur Überwachung von Gangparametern geeignet sind, sowie ein Temperatursensor (6) auch an der Trägerplatte (1) befestigt sind.

4. Verfahren nach einem der Ansprüche 1 bis 3, **dadurch gekennzeichnet, dass** die ganze Vorrichtung mit einem wasserfesten Dichtungsmaterial bedeckt ist.

5. Vorrichtung zur Gleichgewichtsüberwachung, die aufweist
- eine wie eine Schuh-Einlegesohle geformte Trägerplatte (1),
- an der Trägerplatte (1) befestigte Dehnungsmessstreifen (3a-d), und
- eine zentrale Elektronikeinheit (7), die über Leitungen mit den Dehnungsmessstreifen (3a-d) verbunden und an der Trägerplatte (1) befestigt ist,
wobei
- ein Flansch (2), der geeignet ist, eine biegsame Stütze für die Trägerplatte (1) zu liefern, entlang eines Umfangs der Trägerplatte (1) geformt wird, und
- die zentrale Einheit (7) einen auf einem neuronalen Netzwerk basierenden Algorithmus (33) aufweist, der zur Bestimmung - auf der Basis von Signalen der Dehnungsmessstreifen (3a-d) - einer Stellung eines Schwerpunkts eines Körpers geeignet ist und gemäß Anspruch 1 trainiert wird.

6. Vorrichtung nach Anspruch 5, **dadurch gekennzeichnet, dass** die Dehnungsmessstreifen (3a-d) in Dehnungsmessstreifeneinheiten angeordnet sind, die mit der Trägerplatte (1) klebeverbunden sind, wobei jede Dehnungsmessstreifeneinheit einen Trägerkörper und vier Dehnungsmessstreifen mit gleichen Parametern aufweist, wobei die vier Dehnungsmessstreifen auf dem Trägerkörper um 90 Grad zueinander gedreht angeordnet sind.

7. Vorrichtung nach Anspruch 6, **dadurch gekennzeichnet, dass** sie aufweist
- einen ersten Dehnungsmessstreifen (3a), der innerhalb einer Nähe von 10 mm zum Bereich unter dem ersten Mittelfußknochen angeordnet ist, und einen zweiten Dehnungsmessstreifen (3b), der innerhalb einer Nähe von 10 mm zum Bereich unter dem fünften Mittelfußknochen symmetrisch zum ersten Dehnungsmessstreifen (3a) bezüglich einer Mittellinie angeordnet ist, die sich zwischen den zwei äußersten Punkten der Trägerplatte (1) erstreckt, und
- einen dritten Dehnungsmessstreifen (3c) und einen vierten Dehnungsmessstreifen (3d), die symmetrisch bezüglich der Mittellinie in einem Bereich angeordnet sind, der sich 5-15 mm in Richtung der Ferse vom untersten Teil des Fersenbeins befindet.

8. Vorrichtung nach einem der Ansprüche 5-7, **dadurch gekennzeichnet, dass** sie weiter einen dreidimensionalen Beschleunigungssensor (5) und ein dreidimensionales Gyroskop (4), das geeignet ist, Gangparameter zu überwachen, sowie einen Temperatursensor (6) aufweist,
die alle an der Trägerplatte (1) befestigt sind.

## Revendications

1. Procédé pour fabriquer un dispositif pour surveiller l'équilibre, comprenant les étapes consistant à
- fournir une plaque porteuse (1) conformée à la manière d'une semelle intérieure de chaussure,
- fixer des jauges de contraintes (3a-d) sur la plaque porteuse (1),
- fixer une unité électronique centrale (7) sur la plaque porteuse (1), et
- connecter les jauges de contraintes (3a-d) et l'unité électronique centrale (7) les unes aux autres avec des conduits,
- former une bride (2) adaptée à constituer un support flexible pour la plaque porteuse (1) le long d'une circonférence de la plaque porteuse (1), et
- prévoir dans l'unité électronique centrale (7) un algorithme basé sur un réseau neuronal (33), pour déterminer une position d'un centre de gravité d'un corps, fonctionnant sur la base de signaux des jauges de contraintes (3a-d),
dans lequel l'algorithme basé sur un réseau neuronal (33) est soumis à un apprentissage par
- application de forces de pression sur la plaque porteuse (1) à divers emplacements de celle-ci en utilisant des tiges de pression (41) agencées dans un motif en grille pour l'application des forces de pression, et en exerçant des forces de pression d'au moins trois amplitudes différentes avec chaque tige de pression (41),
- mesure des signaux des jauges de contraintes (3a-d) résultant de la déformation provoquée par les forces de pression ; et
- utilisation des données de mesure ainsi obtenues à titre de données d'apprentissage pour l'algorithme basé sur un réseau neuronal (33).

2. Procédé selon la revendication 1, **caractérisé en ce que** l'on sépare un groupe de données de mesure à titre de données de commande, telles que ces données de commande ne sont pas appliquées comme données d'apprentissage, mais l'on teste au contraire la réponse de l'algorithme basé sur un réseau neuronal (33) aux données de commande de temps en temps pendant le processus d'apprentissage, et l'on termine le processus d'apprentissage quand la réponse générée par l'algorithme basé sur un réseau neuronal (33) aux données de commande tombe dans une marge d'erreur.

3. Procédé selon la revendication 1, **caractérisé en ce qu'**un capteur d'accélération tridimensionnel (5) et un gyroscope tridimensionnel (4), tous deux adaptés pour surveiller des paramètres de démarche, ainsi qu'un capteur de température (6), sont également fixés sur la plaque porteuse (1).

4. Procédé selon l'une quelconque des revendications 1 à 3, **caractérisé en ce que** le dispositif entier est couvert avec un matériau d'étanchement étanche à l'eau.

5. Dispositif pour surveiller l'équilibre, comprenant
- une plaque porteuse (1) formée à la manière d'une semelle intérieure de chaussure,
- des jauges de contraintes (3a-d) fixées sur la plaque porteuse (1), et
- une unité électronique centrale (7) connectée avec des conduits aux jauges de contraintes (3a-d) et fixée sur la plaque porteuse (1),
dans lequel
- une bride (2) adaptée à constituer un support flexible pour la plaque porteuse (1) est formée le long d'une circonférence de la plaque porteuse (1), et
- l'unité centrale (7) comprend un algorithme basé sur un réseau neuronal (33) et adapté à déterminer, sur la base de signaux des jauges de contraintes (3a-d), une position d'un centre de gravité d'un corps, et qui subit un apprentissage selon la revendication 1.

6. Dispositif selon la revendication 5, **caractérisé en ce que** les jauges de contraintes (3a-d) sont agencées par unités de jauges de contraintes collées de manière adhésive sur la plaque porteuse (1), dans lequel chaque unité de jauges de contrainte comprend un corps porteur et quatre jauges de contraintes ayant des paramètres identiques, les quatre jauges de contraintes étant agencées sur le corps porteur avec rotation de 90° les unes par rapport aux autres.

7. Dispositif selon la revendication 6, **caractérisé en ce qu'**il comprend
- une première jauge de contraintes (3a) agencée dans une proximité de 10 mm de la région sous le premier os du métatarse, et une seconde jauge de contraintes (3b) agencée dans une proximité de 10 mm de la région sous le cinquième os du métatarse, symétriquement à la première jauge de contraintes (3a) par rapport à une ligne médiane s'étendant entre les deux points les plus extérieurs de la plaque porteuse (1), et
- une troisième jauge de contraintes (3c) et une quatrième jauge de contraintes (3d) agencées symétriquement par rapport à la ligne médiane dans une région située de 5 à 15 mm vers le talon depuis la portion la plus basse de l'os calcanéen.

8. Dispositif selon l'une quelconque des revendications 5 à 7, **caractérisé en ce qu'**il comprend en outre un capteur d'accélération tridimensionnel (5) et un gyroscope tridimensionnel (4), adaptés à surveiller des paramètres de démarche, ainsi qu'un capteur de température (6), qui sont tous fixés sur la plaque porteuse (1).
